(19) Europäisches Patentamt
European Patent Office
Office européen des brevets

(11) Veröffentlichungsnummer: **0 158 278**
**A2**

(12) # EUROPÄISCHE PATENTANMELDUNG

(21) Anmeldenummer: 85104060.0

(22) Anmeldetag: 03.04.85

(51) Int. Cl.[4]: **A 61 K 31/40**

(30) Priorität: 11.04.84 DE 3413672

(43) Veröffentlichungstag der Anmeldung:
16.10.85 Patentblatt 85/42

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Kruse, Hansjörg, Dr.
Feldbergstrasse 70
D-6233 Kelkheim (Taunus)(DE)

(72) Erfinder: Kuch, Heinz, Dr.
Hölderlinstrasse 48
D-6233 Kelkheim (Taunus)(DE)

(54) **Oxindol-Verbindungen zur Behandlung der Epilepsie.**

(57) Oxindol-Verbindungen der Formel I

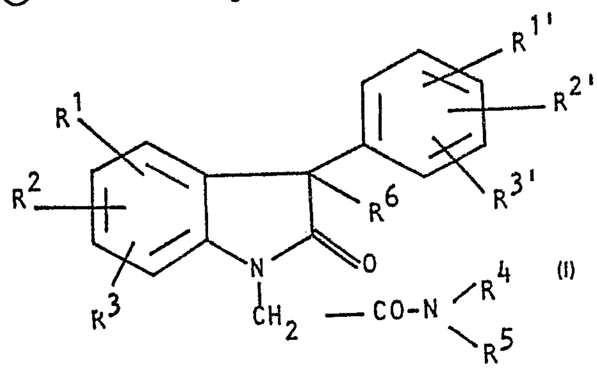

(I)

worin $R^1$, $R^2$, $R^3$, $R^{1'}$, $R^{2'}$, $R^{3'}$, $R^4$, $R^5$ und $R^6$ die angegebenen Bedeutungen haben, sowie deren physiologisch verträgliche Salze, zur Behandlung der Epilepsie und ihre Verwendung zur Herstellung eines Arzneimittels zur Behandlung der Epilepsie werden beschrieben.

EP 0 158 278 A2

0158278

HOECHST AKTIENGESELLSCHAFT HOE 84/F 085 Dr.D/mk

Oxindol-Verbindungen zur Behandlung der Epilepsie

Nach DE-A 31 14 351 (entspr. EP-A 62 887 bzw. US-Patentanmeldung Serial No. 366 321) sind Oxindol-Verbindungen mit neuroanaboler (nootroper) Wirkung bekannt. Es wurde nun überraschenderweise gefunden, daß ein Teil dieser Verbindungen antiepileptische (antikonvulsive) Wirkung aufweist.

Die Erfindung betrifft daher Oxindol-Verbindungen der Formel I

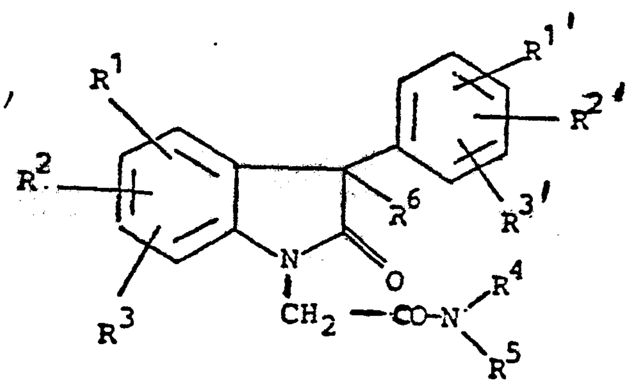

(I)

zur Behandlung der Epilepsie.

In der Formel I bedeuten

$R^1$, $R^2$, $R^3$, $R^{1'}$, $R^{2'}$ und $R^{3'}$ gleich oder verschieden und von einander unabhängig Wasserstoff, Halogen wie Fluor, Chlor oder Brom, geradkettiges oder verzweigtes Alkyl mit 1 bis 4 C-Atomen,

$R^4$ und $R^5$ gleich oder verschieden und von einander unabhängig Wasserstoff, geradkettiges oder verzweigtes Alkyl mit 1 bis 4 C-Atomen; Cyclohexyl, Alkylcyclohexyl mit 1 bis 4 C-Atomen im Alkylteil, Cycloheptyl, Cyclooctyl; Phenyl, das durch Halogen, wie Fluor, Chlor oder Brom, geradkettiges oder verzweigtes Alkyl mit 1 bis 4 C-Atomen, Trifluormethyl, Methylendioxy, Alkoxy mit 1 bis

3 C-Atomen mono- oder disubstituiert sein kann; Phenylalkyl mit 1 bis 3 C-Atomen im Alkylteil, wobei der Phenylkern wie vorstehend bei Phenyl angegeben substituiert sein kann, Hydroxyalkyl mit 1 bis 4 C-Atomen oder $R^4$ und $R^5$ gemeinsam mit dem sie tragenden Stickstoffatom Pyrrolidino, Piperidino, Morpholino oder Phenylpiperazino, wobei der Phenylkern wie vorstehend bei Phenyl ausgeführt substituiert sein kann, und

$R^6$ Wasserstoff, Alkyl mit 1, 2 oder 3 C-Atomen oder Hydroxy bedeuten.

Besonders bevorzugt sind zur Behandlung der Epilepsie Verbindungen der Formel I, worin

$R^{1'}$, $R^{2'}$ und $R^{3'}$ Wasserstoff

$R^1$ Wasserstoff oder Chlor in der 5-Position des Indols;

$R^2$ und $R^3$ Wasserstoff

$R^4$ und $R^5$ gleich oder verschieden und voneinander unabhängig Wasserstoff, Alkyl mit 1, 2 oder 3 C-Atomen, Cyclohexyl, Methylcyclohexyl, Cycloheptyl, Cyclooctyl, Hydroxyalkyl mit 2 oder 3 C-Atomen, Benzyl, Phenethyl, worin die Phenylreste jeweils durch Halogen, wie Fluor oder Chlor, Methylendioxy, Alkoxy mit 1, 2 oder 3 C-Atomen im Phenylkern mono- oder disubstituiert sein können, oder $R^4$ und $R^5$ gemeinsam mit dem sie tragenden Stickstoffatom Piperidino, Pyrrolidino, Morpholino, N-Phenylpiperazino, worin der Phenylkern durch Fluor oder Chlor, Methylendioxy, Alkoxy mit 1, 2 oder 3 Kohlenstoffatome einfach oder zweifach substituiert sein kann, und

$R^6$ Wasserstoff, Methyl oder Hydroxy bedeuten.

Die erfindungsgemäß anzuwendenden Verbindungen der Formel I weisen ein asymetrisches C-Atom auf und treten daher in stereoisomeren Formen auf. Die Erfindung umfaßt die Anwendung

der racemischen Gemische sowie der rechts- und linksdrehenden Enantiomeren.

Soweit die erfindungsgemäß anzuwendenden Verbindungen basischen Charakter haben, umfaßt die Erfindung auch ihre Salze mit pharmazeutisch verträglichen Säuren wie z.B. mit Halogenwasserstoffsäuren, insbesondere Salzsäure, Essigsäure, Äpfelsäure, Weinsäure, Maleinsäure, Fumarsäure, Methansulfonsäure und ähnlichen.

Von ganz besonderer Bedeutung für die Behandlung der Epilepsie ist die Verbindung der Formel I, in welcher die Reste $R^1$ bis $R^6$, $R^{1'}$, $R^{2'}$ und $R^{3'}$ Wasserstoff bedeuten (2-Oxo-3-phenyl-1-indolinessigsäureamid, Formel II)

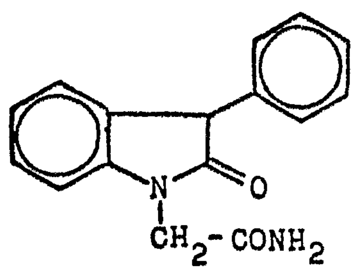

(II)

Die erfindungsgemäß anzuwendenden Verbindungen der Formel I lassen sich nach den in der DE-A 31 14 351 (entspr. EP-A 62 887 bzw. US-Patentanmeldung Serial No. 366 321) beschriebenen Verfahren herstellen.

Die Erfindung betrifft ferner die Verwendung der Verbindungen der Formel I sowie deren Salze zur Herstellung eines Arzneimittels zur Behandlung der Epilepsie.

Die für die Behandlung der Epilepsie geeigneten Verbindungen der Formel I bzw. II oder ihre Salze werden zweckmäßig in üblicher Weise in pharmazeutische Präparate verarbeitet. Für die perorale Anwendung können die Verbindungen der Formel I bzw. II zu Tabletten, Dragees oder Kapseln verarbeitet werden, die gegebenenfalls neben den Wirkstoffen übliche pharmazeutische Trägerstoffe, Verdünnungsmittel und/oder Hilfsstoffe enthalten. Der Wirkstoffgehalt beträgt 1 bis 95 Prozent, vorzugsweise 10 bis 80 Prozent. Als

zugsweise 10 bis 80 Prozent. Als Trägerstoff, Verdünnungsmittel und Hilfsstoffe kommen z.B. Calciumcarbonat, Calciumphosphat, Natriumphosphat, Milchzucker, Maisstärke, Alginate, Gelatine, Aluminium- oder Magnesiumstearat, Talkum oder Siliconöl in Betracht.

Zweckmäßig kann ein solches Arzneimittel in Dosierungseinheiten zubereitet werden, die auf die gewünschte Therapie abgestimmt sind.

Solche Arzneimittel können pro Einzeldosis 1 bis 1000 mg, vorteilhaft 5 bis 500 mg einer Verbindung der Formel I als Wirkstoff enthalten.

Für die parenterale Verabreichung kommt die Anwendung der Wirkstoffe als injizierbare wäßrige oder ölige Suspension in Betracht, die zusätzlich noch Suspendiermittel, wie z.B. Natriumcarboxymethylcellulose, Methylcellulose, Hydroxypropylcellulose, Natriumalginat oder Polyvinylpyrrolidon, Dispergiermittel wie Polyoxyethylenstearat und Konservierungsmittel enthalten können; die öligen Suspensionen können in Erdnuß-, Oliven-, Kokos-, Sesam- oder Paraffinöl vorliegen.

Die Oxindol-Verbindungen der Formel I sind hochwirksam in Testmodellen, die eine antiepileptische Wirkung am Menschen und anderen Säugern erwarten lassen. So verhindern sie die Ausbildung von tonischen und klonischen Krämpfen, die durch eine Reihe von Methoden ausgelöst werden können, so beispielsweise durch Elektroschock, Pentetrazol, Picrotoxin, Bicucullin, Isoniazid oder Nikotin im Dosisbereich zwischen 10 und 1000 mg/kg in Mäusen, Ratten, Goldhamstern und anderen Säugern.

Die Oxindol-Verbindungen der Formel I vermindern auch im Dosisbereich zwischen 10 und 1000 mg/kg die Toxizität des Strychnins. Die bestwirksamen der gemäß der Erfindung anzuwendenden Verbindungen übertreffen in ihrer Wirksamkeit die

zur Zeit am häufigsten verwendeten Antiepileptika bei gleichzeitig verringerten unerwünschten Wirkungen. Die pharmakologische Wirkung wurde in den folgenden Testmodellen geprüft:

1) Elektroschock-induzierte Konvulsionen in Mäusen

Männliche Mäuse (NMRI, Gassner) werden in Testgruppen zu je 10 Tieren eingeteilt und mit der in 1 % Methylcellulose suspendierten Testsubstanz vorbehandelt. Die Kontrollgruppe erhält nur Vehikel.

60 Minuten nach Vorbehandlung wird ein elektrokonvulsiver Schock (12 mA, 50 Hz, 200 msec) via Cornealelektroden verabreicht. Dieser Schock verursacht tonische Extensorkrämpfe in 100 % der Kontrollgruppe. Die $ED_{50}$ ist die Dosis, die die tonischen Krämpfe in 50 % der Tiere verhindert.

2-Oxo-3-phenyl-1-indolinessigsäureamid hat in diesem Modell eine $ED_{50}$ von 214 mg/kg (0.80 mmol/kg). Das zur Zeit am häufigsten verwendete Antiepileptikum Natrium-2-propylvalerat hat hier eine $ED_{50}$ von 259 mg/kg (1.80 mmol/kg).

2) Verhinderung von durch Pentetrazol erzeugten Krämpfen in Mäusen

Männliche Mäuse werden in Testgruppen zu je 10 Tieren wie unter 1) beschrieben vorbehandelt. 60 min nach Vorbehandlung wird jeweils eine Dosis von 125 mg/kg Pentetrazol subkutan verabreicht. Diese Dosis erzeugt tonisch-klonische Krämpfe in 95 % der Kontrolltiere. Während einer 30minütigen Beobachtungszeit wird die Konvulsionsrate bestimmt, wobei die Tiere einzeln in Glaszylindern gehalten werden. Die $ED_{50}$ ist die Dosis, die die Rate der Konvulsionen auf 50 % verringert.

2-Oxo-3-phenyl-1-indolinessigsäureamid hat in diesem Modell eine $ED_{50}$ von 176 mg/kg (0.66 mmol/kg) gegen tonische Krämpfe, Natrium-2-propylvalerat 158 mg/kg (1.10 mmol/kg).

3) Verhinderung von durch Picrotoxin erzeugten Krämpfen in Mäusen

Männliche Mäuse (NMRI, Gassner) werden in Testgruppen zu 10 Tieren eingeteilt und wie unter 1) beschrieben vorbehandelt. Nach 60 Minuten Vorbehandlung wird jeweils eine Dosis von 15 mg/kg Picrotoxin subkutan verabreicht, was in 95 % der Kontrolltiere tonisch-klonische Konvulsionen erzeugt. Während einer 30minütigen Beobachtungsperiode wird die Zahl der Konvulsionen bei den einzelnen Tieren bestimmt. Die $ED_{50}$ ist diejenige Dosis, die die Zahl der Konvulsionen um 50 % vermindert im Vergleich zur Kontrollgruppe. 2-Oxo-3-phenyl-indolinessigsäureamid hat in diesem Modell eine $ED_{50}$ von 78.6 mg/kg (0.30 mmol/kg), Natrium-2-propylvalerat von 75.4 mg/kg (0.52 mmol/kg).

4) Verhinderung der durch Bicucullin induzierten Krämpfe in Mäusen

Die Durchführung des Versuchs erfolgte nach dem in 3) beschriebenen Verfahren, jedoch wurde Picrotoxin durch 5 mg/kg Bicucullin subkutan ersetzt.

2-Oxo-3-phenyl-1-indolinessigsäureamid hat in diesem Modell eine $ED_{50}$ von 426 mg/kg (1.60 mmol/kg), Natrium-2-propylvalerat von 362 mg/kg (2.51 mmol/kg).

5) Verhinderung der durch Isoniazid verursachten Krämpfe in Mäusen

Die Durchführung des Versuchs erfolgt nach dem in 3) beschriebenen Verfahren, jedoch wird Picrotoxin durch 600 mg/kg Isoniazid subkutan ersetzt.

2-Oxo-3-phenyl-1-indolinessigsäureamid hat in diesem Versuch eine $ED_{50}$ von 500 mg/kg (1.88 mmol/kg), Natrium-2-propylvalerat von 494 mg/kg (3.43 mmol/kg).

6) Verhinderung der durch Nikotin erzeugten Krämpfe in Mäusen

Die Durchführung des Versuchs erfolgt nach dem in 3) beschriebenen Verfahren, jedoch wird Picrotoxin durch 1 mg/kg Nikotin subkutan ersetzt. Darüber hinaus wird die Todesrate in der 30minütige Beobachtungsphase bestimmt. Die $ED_{50}$ ist hier die Dosis, die die Letalität um 50 % vermindert.

2-Oxo-3-phenyl-indolinessigsäureamid hat in diesem Modell bezüglich der Konvulsionen eine $ED_{50}$ von 118 mg/kg (Natrium-2-propylvalerat: 168 mg/kg) und bezüglich der Letalität von 119 mg/kg (Natrium-2-propyl-valerat : 186 mg/kg).

7) Verhinderung der Strychnin-Letalität in Mäusen

Die Vorbehandlung erfolgte wie unter 1) beschrieben. 60 min nach Vorbehandlung wurde eine Dosis von 2 mg/kg Strychnin subkutan verabreicht, was in der Kontrollgruppe 95 % der Tiere tötet. In einem 30minütigem Beobachtungszeitraum wird die Todesrate bestimmt. Die $ED_{50}$ ist die Dosis, die die Todesrate um 50 % vermindert.

2-Oxo-3-phenyl-1-indolinessigsäureamid hat in diesem Versuch eine $ED_{50}$ von 376 mg/kg (1.41 mmol/kg), Natrium-2-propylvalerat von 307 mg/kg (2.13 mmol/kg).

8) Verhinderung der durch Pentetrazol verursachten Krämpfe in Goldhamstern

Die Durchführung des Versuchs erfolgt analog der unter 2) beschriebenen Methode, jedoch werden anstelle der Mäuse pro

Testgruppe 10 Goldhamster (Zuchtkolonie HOECHST) verwendet.

2-Oxo-3-phenyl-1-indolinessigsäureamid hat in diesem Modell eine $ED_{50}$ von 127 mg/kg (0.48 mmol/kg), Natrium-2-propylvalerat von 536 mg/kg (3.7 mmol/kg).

9) Verhinderung der durch Elektroschock ausgelösten Krämpfe an Ratten

Die Versuchsdurchführung entspricht der unter 1) beschriebenen, jedoch wurden anstelle der Mäuse männliche Ratten (Wistar, Ivanovas, 6 - 10 pro Testgruppe) verwendet.

Der elektrokonvulsive Schock, der hier verwendet wurde, hatte folgende Charakteristik: 30 mA, 50 Hz, 200 msec.

In diesem Modell hat 2-Oxo-3-phenyl-1-indolinessigsäureamid eine $ED_{50}$ von 364 mg/kg (1.37 mmol/kg), Natrium-2-propylvalerat 450 mg/kg (3.13 mmol/kg).

10) Wirkdauer gegen durch Elektroschock ausgelöste Krämpfe in Mäusen

160 männliche Mäuse (NMRI, Ivanovas) wurden in Testgruppen zu je 10 Tieren aufgeteilt. Eine maximal effektive Dosis von 2-Oxo-3-phenyl-1-indolinessigsäureamid (300 mg/kg) per os bzw. Natrium-2-propylvalerat (500 mg/kg) per os wurden verabreicht. Zu unterschiedlichen Zeiten nach der Vorbehandlung von 0.5 bis 8 Stunden wurde jeweils einer Testgruppe via Cornealelektrode ein Elektroschock (12 mA, 50 Hz, 200 msec), der in der Kontrollgruppe in 100 % der Extensorkrämpfe auslöst, verabreicht. Die Halbwertszeit ist diejenige Zeit, nach der noch 50 % der Tiere gegen diese Krämpfe geschützt sind. Sie beträgt für 2-Oxo-3-phenyl-1-indolinessigsäureamid mehr als 8 Stunden, für Natrium-2-propylvalerat 5.5 Stunden.

Die beschriebenen Ergebnisse in den verschiedenen Versuchsmodellen lassen eine starke Wirksamkeit bei der Epilepsie am Menschen erwarten.

Die im folgenden aufgeführten Beispiele sollen die Herstellung geeigneter Präparate für die erfindungsgemäße Anwendung erläutern.

Beispiel 1

Herstellung eines erfindungsgemäß verwendeten Mittels zur oralen Anwendung in der Behandlung der Epilepsie:

1000 Tabletten, die je 50 mg 2-Oxo-3-phenyl-1-indolin-essigsäureamid enthalten, werden wie folgt hergestellt:

Rezeptur

| | |
|---|---|
| 2-Oxo-3-phenyl-1-indolinessigsäureamid | 50 g |
| Maisstärke | 50 g |
| Gelatine | 40 g |
| Mikrokristalline Cellulose | 50 g |
| Magnesiumstearat | 10 g |

2-Oxo-3-phenyl-1-indolinessigsäureamid wird mit einer Gelatinlösung gemischt, die Mischung wird getrocknet und zu einem Granulat vermahlen. Maisstärke, mikrokristalline Cellulose und Magnesiumstearat werden mit dem Granulat vermischt. Aus der Mischung werden 1000 Tabletten gepreßt, die 50 mg der aktiven Verbindung enthalten und zur Behandlung der Epilepsie verwendet werden können.

Beispiel 2

Gelatinkapseln, die je 100 mg 2-Oxo-3-phenyl-1-indolin-essigsäureamid enthalten, werden wie folgt hergestellt: Eine Mischung von 2-Oxo-3-phenyl-1-indolin-essigsäureamid (100 mg), Magnesiumstearat (2 mg) und Lactose (135 mg) wird in je eine Gelantinekapsel eingefüllt. Die Kapseln können zur peroralen Anwendung in der Behandlung der Epilepsie verwendet werden.

PATENTANSPRÜCHE: HOE 84/F 085

1) Verbindungen der Formel I, in welcher

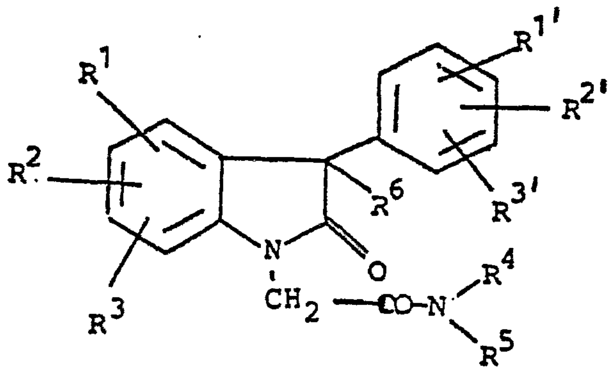

(I)

$R^1$, $R^2$, $R^3$, $R^{1'}$, $R^{2'}$ und $R^{3'}$ gleich oder verschieden und von einander unabhängig Wasserstoff, Halogen geradkettiges oder verzweigtes Alkyl mit 1 bis 4 C-Atomen

$R^4$ und $R^5$ gleich oder verschieden und von einander unabhängig Wasserstoff, geradkettiges oder verzweigtes Alkyl mit 1 bis 4 C-Atomen; Cyclohexyl, Alkylcyclohexyl mit 1 bis 4 C-Atomen im Alkylteil, Cycloheptyl, Cyclooctyl; Phenyl, das durch Halogen, geradkettiges oder verzweigtes Alkyl mit 1 bis 4 C-Atomen, Trifluormethyl, Methylendioxy, Alkoxy mit 1 bis 3 C-Atomen mono- oder disubstituiert sein kann; Phenylalkyl mit 1 bis 3 C-Atomen im Alkylteil, wobei der Phenylkern wie vorstehend bei Phenyl angegeben substituiert sein kann, Hydroxyalkyl mit 1 bis 4 C-Atomen oder $R^4$ und $R^5$ gemeinsam mit dem sie tragenden Stickstoffatom Pyrrolidino, Piperidino, Morpholino oder Phenylpiperazino, wobei der Phenylkern wie vorstehend bei Phenyl ausgeführt substituiert sein kann, und

$R^6$ Wasserstoff, Alkyl mit 1, 2 oder 3 C-Atomen oder Hydroxy bedeuten, sowie von deren physiologisch verträgliche Salze

zur Behandlung der Epilepsie.

2) 2-Oxo-3-phenyl-1-indolinessigsäureamid gemäß Anspruch 1 zur Behandlung der Epilepsie.

3) Verbindung der Formel I sowie deren physiologisch verträgliche Salze zur oralen oder parenteralen Behandlung der Epilepsie.

4) Pharmazeutisches Präparat zur Behandlung der Epilepsie, dadurch gekennzeichnet, daß es eine Verbindung gemäß Anspruch 1 kombiniert mit pharmazeutischen Hilfsstoffen und Trägerstoffen enthält.

5) Verwendung einer Verbindung gemäß Anspruch 1 zur Herstellung eines Arzneimitels zur Behandlung der Epilepsie.

6) Verwendung von 2-Oxo-3-phenyl-1-indolinessigsäureamid gemäß Anspruch 2 zur Herstellung eines Arzneimittels zur Behandlung der Epilepsie.

7) Verfahren zur Behandlung der Epilepsie, dadurch gekennzeichnet, daß man eine wirksame Menge einer Verbindung gemäß Anspruch 1 verabreicht.